# EUROPÄISCHE PATENTANMELDUNG

(11) **EP 1 419 995 A1**
(43) Veröffentlichungstag der Anmeldung: **19.05.2004**
(21) Anmeldenummer: 02025529.5
(22) Anmeldetag: 13.11.2002
(51) Int. Cl.: C02F 3/28, C02F 1/24

(54) **Anaerobfermenter**

(71) Anmelder: Von Nordenskjöld, Reinhart, Dr.-Ing., D-85658 Egmating-Münster (DE)
(72) Erfinder: Von Nordenskjöld, Reinhart, Dr.-Ing., D-85658 Egmating-Münster (DE)
(74) Vertreter: Habersack, Hans-Jürgen, Dipl.-Ing.

(57) **Zusammenfassung**

Die Erfindung betrifft eine Vorrichtung (1) zum anaeroben Fermentieren von Stoffen (A, B) mit einem Vorversäuerer (2), in dem die Stoffe (A, B) einer Vorversäuerung unterliegen, einem Fermenter (3a, 3b), in dem die vorversäuerten Stoffe (A, B) fermentieren und Überführmittel (5a, 5b, 5c, 5d, 5e, 10, 11, 12, 14, 21) zum Überführen der Stoffe (A, B) aus dem Vorversäuerer (2) in den Fermenter (3a, 3b). Die Vorrichtung ist dadurch asugezeichnet, dass die Überführmittel (5a, 5b, 5c, 5d, 5e, 10, 11, 12, 14, 21) zum selektiven Überführen von ausreichend vorversäuerten Stoffen (A, B) ausgebildet sind.

Weiterhin betrifft die Erfindung ein Verfahren zum anaeroben Fermentieren von Stoffen (A, B) mit einer Vorversäuerung, bei der die Stoffe (A, B) mit einem Vorversäuerer (2) vorversäuert werden, einem Fermentieren, bei dem die vorversäuerten Stoffe (A, B) in einem Fermenter (3a, 3b) fermentieren, und einem Überführen, bei dem Stoffe (A, B) aus dem Vorversäuerer (2) in den Fermenter (3a, 3b) überführt werden, wobei selektiv die ausreichend vorversäuerten Stoffe überführt werden.

## Beschreibung

Die Erfindung betrifft eine Vorrichtung und ein Verfahren zum anaeroben Fermentieren von Stoffen.

Eine Vorrichtung und ein Verfahren gemäß dem Oberbegriff von Anspruch 1 und von Anspruch 6 ist aus der DE 198 04 007 bekannt. Dieses Dokument betrifft die Behandlung von organisch belastetem Fluid, wobei Gas in der Fermentationsstufe generiert wird. In der Praxis hat sich gezeigt, dass die durchschnittliche Aufenthaltsdauer der belasteten Fluide in der Vorversäuerungsstufe ca. 15 Stunden und ein Gesamtprozess ca. 30 Stunden beträgt. In dieser Zeit ist das Prozesswasser ausreichend vorversäuert, so dass es in den Fermenter überführt werden kann, ohne dass die Gefahr besteht, dass in dem Fermenter noch eine weitere Versäuerung erfolgen würde, was für den Fermentationsprozess, insbesondere durch Schädigung der Methanbakterien auf Grund niedriger pH-Werte, sehr nachteilig wäre. Üblicherweise muss in der Vorversäuerung ein pH-Wert von 6,0 oder darunter erreicht werden. Die Vorversäuerung dient dazu komplexe Kohlenstoffverbindungen aufzuschließen, da die Fermentationsbakterien nur einfache Kohlenstoffverbindungen nutzen können.

Bei der Fermentation wird Biogas gewonnen, das eine Mischung aus Methan und Kohlendioxid darstellt und das zur Energiegewinnung benutzt werden kann.

Aus der Praxis sind weiterhin Feststoffreaktoren bekannt, in denen gemeinsam Vorversäuerung und Fermentation zur Gewinnung von Biogas stattfinden. In derartigen Reaktoren können feste bis pastöse Stoffe behandelt werden, deren Aufenthaltszeit Wochen beträgt, bis ein ausreichender Gesamtprozess stattgefunden hat.

Bei Brauereien fallen z. B. flüssige belastete Prozesswasser aus z. B. Reinigungsvorgängen und als festere, organische Stoffe z.B. Hefen und Treber an. Bei Fruchtsaftgewinnungsanlagen fallen neben dem Prozesswasser auch Pressreste wie feste Schalenstücke an. Bei derartigen und ähnlichen Produktionsstätten, bei denen sowohl organisch belastete Flüssigkeiten als auch organische Reststoffe anfallen, sind normalerweise im Wesentlichen wegen der inkompatiblen Vorversäuerungszeiten daher beide, d. h. zwei unabhängige Reaktoren notwendig, um die Stoffe zur Biogasgewinnung zu verwerten. Dies ist jedoch recht kostspielig und kompliziert.

Aufgabe der vorliegenden Erfindung ist es daher, eine Vorrichtung und ein Verfahren zu schaffen, mit der eine kostengünstigere, einfachere Alternative zur Biogasgewinnung aus flüssigen und festen Stoffen realisiert werden kann.

Diese Aufgabe wird gelöst durch eine Vorrichtung mit den Merkmalen von Anspruch 1 und ein Verfahren mit den Merkmalen von Anspruch 8.

Bevorzugte Ausführungsformen sind in den jeweiligen Unteransprüchen offenbart.

Bei der erfindungsgemäßen Vorrichtung sind Mittel zum Überführen von Stoffen in den Fermenter vorgesehen, mit denen es möglich ist, durch selektive Prozessführung alle Stoffe ausreichend vorversäuert zu überführen. Hierbei werden noch nicht ausreichend vorversäuerte, in der Regel festere, wenig aufgeschlossene Stoffe im Vorversäuerer zur ausreichenden Vorversäuerung belassen.

Dementsprechend betrifft das erfindungsgemäße Verfahren ein solches, bei dem nur ausreichend vorversäuerten Stoffe überführt werden.

Auf diese Art und Weise ist es möglich sowohl flüssige, bzw. weitgehend aufgeschlossene, als auch feste oder pastöse, weniger aufgeschlossene Stoffe in einen Vorversäuerer zu geben und anschließend ausreichend vorversäuert einem gemeinsamen Fermenter zuzuführen.

Für die selektive Überführung können verschiedene Stoffeigenschaften zunutze gemacht werden, die die ausreichend vorversäuerten Stoffe von den noch nicht ausreichend vorversäuerten Stoffen unterscheiden.

Während z.B. ausreichend vorversäuerte, feste oder pastöse Stoffe durch den Vorversäuerungsaufschluss aufgeweicht sind und sich im Fluid lösen, sind die nicht ausreichend vorversäuerten Feststoffe in der Regel grobkörnig bzw. sedimentierend. Daher kann eine Selektion beispielsweise durch Sieben oder Sedimentieren erfolgen, wobei z. B. das Sieb so ausgelegt sein muss, dass die noch nicht ausreichend vorversäuerten Feststoffe in dem Sieb hängen bleiben und die flüssigen und aufgeweichten Stoffe durch das Sieb hindurchtreten können.

Weiterhin hat sich gezeigt, dass sich die noch nicht ausreichend vorversäuerten Feststoffe im Vorversäuerer an dessen Boden absetzen, wenn keine Umwälzung oder kein Rühren in dem Vorversäuerer stattfindet. Das bedeutet, dass sich bei Abstellen der Umwälzung oder des in der Regel im Vorversäuerer stattfindenden Rührens die nicht ausreichend vorversäuerten Stoffe im unteren Bereich sammeln, wohingegen sich die ausreichend vorversäuerten Stoffe in einem oberen Bereich sammeln, wodurch eine Selektion von ausreichend vorversäuerten Stoffen durch eine Entnahme von Stoffen im oberen Bereich des Vorversäuerers ermöglicht wird, in dem man die Weiterleitung in den Fermenter nur in den "Ruhephasen" durchführt.

Weiterhin hat sich gezeigt, dass durch eine Flotation, z.B. durch Einblasen von Luft oder Gas in den Vorversäuerer von unten die Feststoffe bevorzugt an der Oberfläche aufschwemmen, so dass sich auch hier eine Trennung von noch nicht ausreichend vorversäuerten Stoffen von den ausreichend vorversäuerten Stoffen ergibt, so dass eine Selektion beim Überführen durch eine Entnahme von Stoffen aus dem unteren Bereich des Vorversäuerers möglich ist.

Eine bevorzugte Ausführungsform bei sehr groben Ausgangsmaterialien wie Schalen, Körner o.ä. beinhaltet eine Vorbehandlung bevorzugt mechanische Vorzerkleinerung der Feststoffe mit einem Häcksler oder einer Mühle oder ähnlichem. Eine Vorbehandlung, insbesondere mechanische Vorzerkleinerung, erleichtern den Aufschluss der festen Stoffe in dem Vorversäuerer.

Bevorzugte Ausführungsformen der erfindungsgemäßen Vorrichtung und des erfindungsgemäßen Verfahrens werden anhand der beiliegenden Figuren erläutert. Dabei zeigt:
- Figur 1: eine dreidimensionale perspektivische Ansicht einer erfindungsgemäßen Vorrichtung,
- Figur 2: eine schematische Schnittansicht der erfindungsgemäßen Vorrichtung,
- Figur 3: eine schematische Schnittansicht einer anderen erfindungsgemäßen Ausführungsform der Erfindung und
- Figur 4: eine Schnittansicht einer weiteren Ausführungsform der Erfindung.

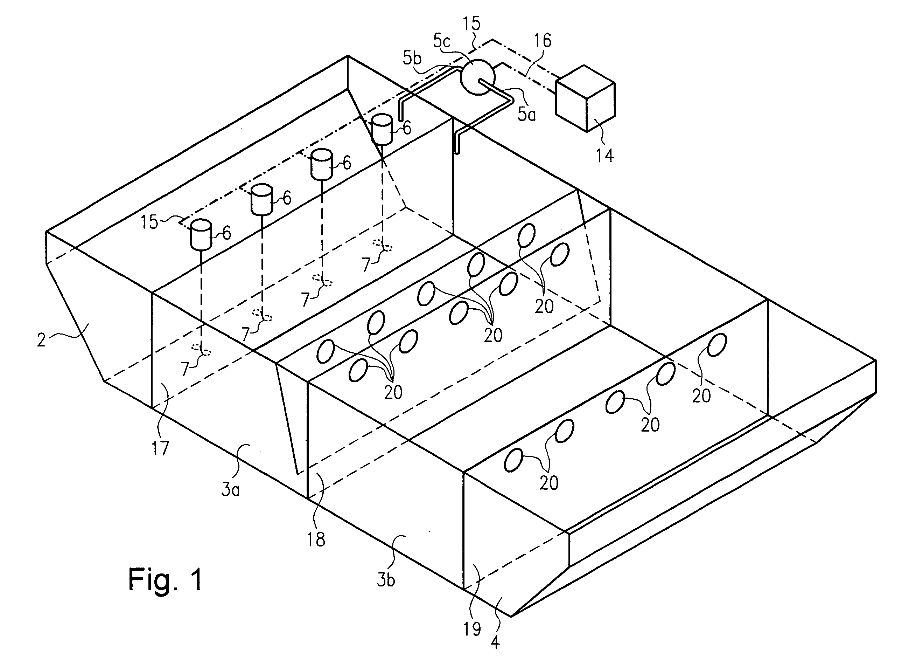

In Figur 1 ist in einer schematischen dreidimensionalen Darstellung eine Vorrichtung zur Vorversäuerung und anaeroben Fermentieren von Stoffen gezeigt. Die Vorrichtung umfasst einen Vorversäuerer 2, einen Fermenter mit einer Hauptlaststufe 3a und einer Schwachlaststufe 3b und einer Nachklärstufe 4. Zur Durchführung der anaeroben Fermentierung ist die Hauptlaststufe 3a und die Schwachlaststufe 3b beispielsweise von einer Plane zum Auffangen und Speichern der anfallenden Gase abgedeckt, die in Figur 1 aus Gründen der Übersichtlichkeit nicht dargestellt ist. Diese Plane kann auch die Vorversäuerung 2 mit abdecken, z. B. wenn die Flotierung mittels Gas oder auch Gaskreislaufs erfolgt (nicht dargestellt).

Zwischen dem Vorversäuerer 2 und der Hauptlaststufe 3a ist eine Trennwand 17, zwischen der Hauptlaststufe 3a und der Schwachlaststufe 3b eine Doppeltrennwand 18 sowie zwischen der Schwachlaststufe 3b und der Nachklärstufe 4 eine Trennwand 19 eingezogen, die verstellbar oder feststehend sein können.

Im Bereich des Vorversäuerers 2 können Rührwerke 6, 7 angeordnet sein, mit denen im Bereich des Vorversäuerers 2 eine Umwälzung durchgeführt werden kann. Die Rührwerke 6, 7 sind von einer Steuereinrichtung 14 über Signal oder Stromversorgungsleitungen 15 ansteuerbar. Die Steuereinrichtung 14 kann über eine Signal- oder Stromleitung 16 ebenfalls mit einer Pumpe 5c verbunden sein, mit der über Rohrleitungen 5a und 5b ein Überführen aus dem Vorversäuerer 2 in die Hauptlaststufe 3a stattfinden kann. Hierbei ist das Ende eines Stutzens und einer Rohrleitung 5b im Bereich des Vorversäuerers 2 im oberen Bereich des Vorversäuerers 2, d.h. beispielsweise oberhalb der Mitte entlang der Höhe des Vorversäuerers 2 angeordnet. Sowohl die Hauptlaststufe 3a als auch die Schwachlaststufe 3b können weiter nicht dargestellte Unterteilungswände umfassen. Die Doppeltrennwand 18 zwischen der Hauptlaststufe 3a und der Schwachlaststufe 3b sowie die Trennwand 19 zwischen der Schwachlaststufe 3b und dem Nachklärer 4 können Durchlässe 20 oder Überläufe umfassen, mit denen Stoffe aus der jeweiligen Stufe in die nachfolgende Stufe übertreten können.

Das in Figur 1 dargestellte Becken kann in den Erdboden eingelassen sein, der jedoch aus Gründen der Übersichtlichkeit in Figur 1 nicht dargestellt ist.

Während in Fig. 1 der Vorversäuerer 2, der Fermenter 3a,3b sowie die Nachklärung 4 in einem Becken untergebracht sind, können diese auch jeweils einzeln in einem Becken oder Tank angeordnet sein.

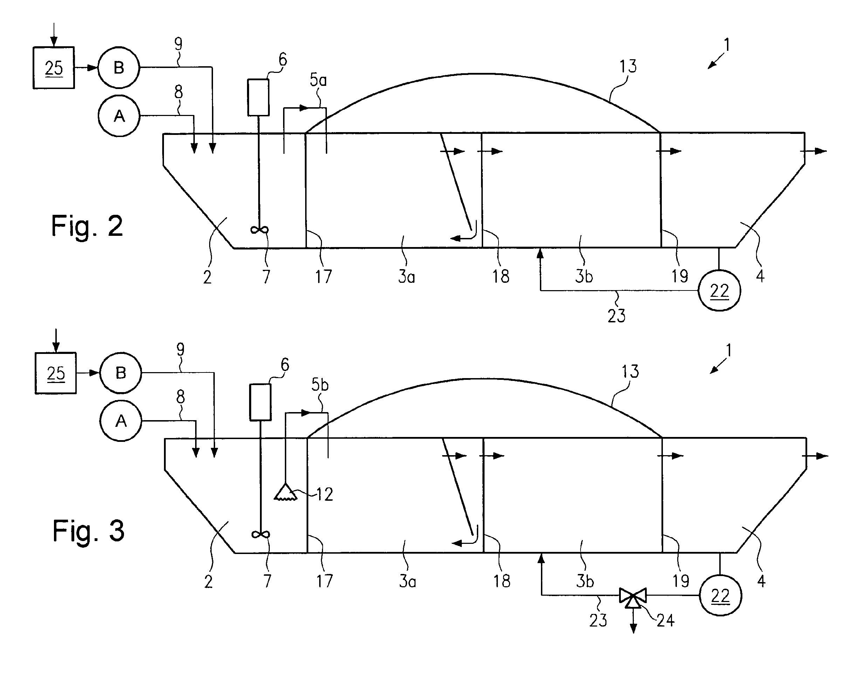

In Figur 2 ist eine schematische Schnittansicht der Vorrichtung aus Figur 1, bei der die Plane 13 dargestellt ist, die die Hauptlaststufe und die Schwachlaststufe abdeckt. Unter der Plane 13 kann sich das erzeugte Biogas sammeln, so dass sich die Plane, wie in Figur 2 dargestellt, aufwölbt.

Weiterhin ist in Figur 2 die Einleitung der Stoffe A und B über die Zuführmechanismen 8 und 9 bzw. für B alternativ über eine Vorbehandlung 25 schematisch dargestellt. Während der flüssige Stoff A in der Regel durch eine Rohrleitung zugeführt wird, kann der feste Stoff B über ein Förderband oder über Behälter oder ähnliches zugeführt werden. Falls der feste Stoff B in einer pastösen oder aufgeschwemmten Form vorliegt, kann er auch über eine Rohrleitung 9 zugeführt werden. In Figur 2 ist auch eine mögliche Vorbehandlung 25 der festen Stoffe B angegeben.

In Figur 3 ist eine andere Ausführungsform der Vorrichtung 1 schematisch dargestellt. Hier ist ein Sieb 12 am Eingang einer Rohrleitung 5b angeordnet, die zum Überführen aus dem Vorversäuerer 2 in die Hauptlaststufe 3a dient. Während das Sieb 12 hier in einer etwa mittleren Position bezüglich der senkrechten Höhe des Vorversäuerers 2 angeordnet ist, kann es auch weiter oben oder weiter unten angeordnet sein. Das Sieb 12 muss eine derartige Maschenweite oder Durchlassöffnungsgröße haben, so dass Feststoffe B, die in den Vorversäuerer eingeführt werden, nicht hindurchtreten können, solange sie nicht ausreichend vorversäuert sind.

Anstelle einer Rohrleitung 5b und einem Sieb 12 als überführmittel kann auch ein Sieb oder ein Rechen in einem Überlauf oder nur ein Überlauf mit automatisch verschließbaren Ventil angeordnet sein, durch den Stoffe aus dem Vorversäuerer 2 in die Hauptlaststufe 3 überlaufen. Ein derartiger Überlauf kann beispielsweise bei dem oberen Ende der Trennwand 17 angeordnet werden und evtl. verschließbar sein. Die beschriebenen Überläufe können auch im mittleren oder unteren Teil der Trennwand 17 angeordnet sein.

Anstelle eines Siebs 12 kann auch jede andere geeignete Einrichtung zum Trennen von groben, strukturierten Stoffen von weichen oder flüssigen Stoffen, wie beispielsweise eine Zwischenklärstufe vorgesehen sein. Aus einer derartige Zwischenklärstufe würden die Feststoffe in den Vorversäuerer 2 zurückgefördert, so dass sie einer weiteren Vorversäuerung unterzogen werden.

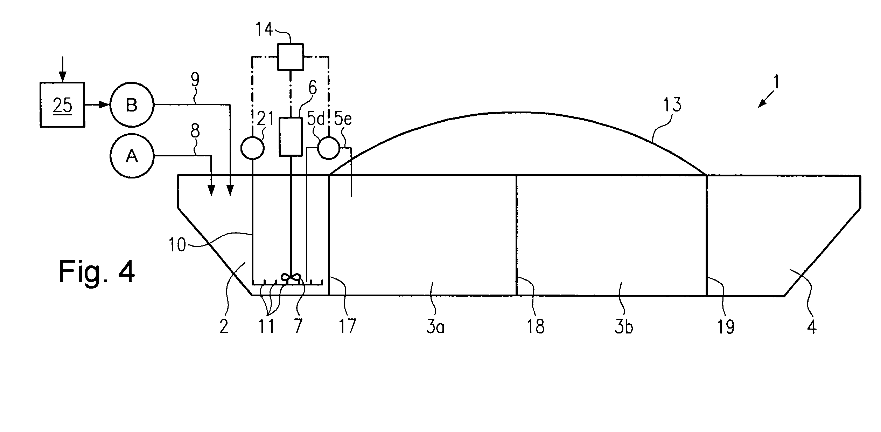

Figur 4 zeigt eine weitere Ausführungsform der Erfindung. Hierbei ist ein Rohr 5e als Überführmittel vorgesehen, dessen Eingangsstutzenende im unteren Bereich des Vorversäuerers 2 liegt. Weiterhin ist eine Flotationseinrichtung 10, 11 vorgesehen, mit der Luft oder Gas, das durch eine Rohrleitung 10 zugeführt wird, über Austrittsöffnungen 11 im Bodenbereich des Vorversäuerers 2 eingeleitet werden kann.

Die Pumpe 5d ist über eine Signal-, Druckluft-, Hydraulik- oder Stromleitung mit einer Steuereinrichtung 14 verbunden, die die Pumpe 5d oder ein automatisches Ventil (nicht dargestellt) ansteuern kann. Weiterhin ist die Steuereinrichtung 14 mit einem optionalen Rührwerk 6,7 und/oder der Flotationseinrichtung 10, 11, 21 über eine oder mehrere Signal,-Druckluft-, Hydraulik- oder Stromleitung verbunden und kann diese ansteuern.

Eine erste Ausführungsform eines erfindungsgemäßen Verfahrens soll anhand der Figuren 1 und 2 erläutert werden.

Über Rohrleitungen 8 wird beispielsweise ein Brauereiabwasser (Prozesswasser) in den Bereich des Vorversäuerers 2 zugeführt. Der weiterhin bei der Brauerei anfallenden Treber, Hefen und Filterreste können roh, ganz oder teilweise vorbehandelt über geeignete Fördermittel, wie Rohrleitungen oder Förderbänder 9 oder ähnliches in den Bereich des Vorversäuerers 2 als Feststoffe B zugeführt werden.

In diesem Bereich erfolgt eine Vergärung durch acidogene Bakterien zu hauptsächlich organischen Säuren, Wasserstoffkohlendioxid sowie niedrigen Alkoholen. Hierbei wird ein pH-Wert von etwa 6 und darunter oder 5,5 und darunter erreicht.

Zur Unterstützung der Durchmischung werden beispielsweise ein oder mehrere Rührwerke 6, 7 im Bereich der Vorversäuerung 2 eingesetzt.

Die Stoffe A und B werden kontinuierlich oder stoßweise in den Bereich des Vorversäuerers 2 gegeben.

Aus dem Vorversäuerer 2 muss kontinuierlich oder in Intervallen eine Überführung von Stoffen in den Hauptlastreaktor 3a geschehen. Hierzu werden mittels der Steuereinrichtung 14 z. B. die Rührwerke 6 oder 7 oder andere Umwälzeinrichtungen abgeschaltet. Nach einiger Zeit (einige Minuten bis einige zehn Minuten) setzen sich die Feststoffe im unteren Bereich des Vorversäuerers 2 ab, die in der Regel noch nicht ausreichend vorversäuert sind. Im oberen Bereich des Reaktors sammeln sich die ausreichend vorversäuerten flüssigen Stoffe sowie aufgeweichten und dabei ausreichend vorversäuerten Feststoffe an. Nach dem Absetzen im Bereich des Vorversäuerers 2, was durch ein Abschalten z. B. der Rührwerke 6, 7 hervorgerufen wird, wird eine Pumpe 5c mittels der Steuereinrichtung 14 eingeschaltet, die Stoffe über die Rohrleitungen 5a und 5b aus dem oberen Bereich des Vorversäuerers 2 in die Hauptlaststufe 3a überführt, oder die Steuereinrichtung 14 öffnet das alternativ eingebaute Ventil.

In der Hauptlaststufe 3a und der Schwachlaststufe 3b fermentieren bzw. methanisieren die vorversäuerten Stoffe unter Bildung von Biogas und die aus der Schwachlaststufe 3b austretenden Stoffe werden in einer Nachklärstufe 4 nachgeklärt. Die nachgeklärten Stoffe können mittels Pumpe 22 und Rohrleitung 23 dem Prozess, z.B. in die Schwachlaststufe 3b wieder zugeführt werden oder mittels Ventil 24 gesteuert als Überschussschlamm entnommen werden.

Der Treber und die Filterreste können in der Vorbehandlung 25 mechanisch vorzerkleinert oder andersartig aufbereitet werden, so dass sich z. B. eine voreingestellte maximale Feststoffkomgröße ergibt. Dies ist zur kontrollierten Prozesssteuerung sowie zur beschleunigten Vorversäuerung der Feststoffe vorteilhaft.

Anhand von Figur 3 soll eine weitere Ausführungsform des Verfahrens erläutert werden. Die Zugabe von Stoffen A, B zum Vorversäuerer 2 sowie die Fermentierung und Nachklärung ist dieselbe, wie die in Bezug auf die Figuren 1 und 2 beschriebene Verfahrensweise.

Bei diesem Verfahren werden kontinuierlich oder in Intervallen Stoffe aus dem Vorversäuerer 2 in die Hauptlaststufe 3a mit einer Rohrleitung 5b und einer nicht dargestellten Pumpe durch ein Sieb 12 überführt. Das Sieb 12 hält die noch nicht ausreichend vorversäuerten Feststoffe zurück und lässt die ausreichend vorversäuerten flüssigen und aufgeweichten Stoffe durch.

Die Entnahme aus dem Vorversäuerer 2 kann hierbei in einer mittleren Position, wie in Figur 3 dargestellt, oder auch weiter oben oder weiter unten erfolgen.

Weiterhin können Mittel zum Freihalten des Siebes 12, wie etwa Schieber, vorgesehen sein, die in geeigneten Abständen sich auf dem Sieb 12 ansammelndes Gut entfernen. Dazu kann auch eine Einrichtung vorgesehen sein, die in dem Sieb 12 eine Gegenströmung erzeugt, um so sich ansammelndes Gut von dem Sieb 12 zu entfernen.

Die Umwälzeinrichtung, hier die Rührwerke 6, 7, können während des Überführens eingeschaltet oder ausgeschaltet sein.

Anhand von Figur 4 soll eine weitere Ausführungsform eines Verfahrens erläutert werden.

Hierbei ist die Einbringung der Stoffe A, B und die Fermentierung bzw. Nachklärung dieselbe, wie die in Bezug auf die Figuren 1 und 2 beschriebene.

Zum Überführen von Stoffen aus dem Vorversäuerer 2 in die Hauptlaststufe 3a wird mit der Steuereinrichtung 14 beispielsweise eine Pumpe 21 angesteuert, mit der Luft oder Gas durch eine Rohrleitung 10 in den unteren Bereich des Vorversäuerers gepumpt wird. Dort tritt die Luft oder das Gas durch Austrittsöffnungen 11 aus, so dass eine Flotation im Vorversäuerer 2 eintritt. Hierbei werden die nicht ausreichend vorversäuerten Feststoffe nach oben aufgeschwemmt. Während dessen wird mit der Steuereinrichtung 14 eine Pumpe 5d in Betrieb gesetzt, die über Rohrleitungen 5e Stoffe aus dem Vorversäuerer 2 in die Hauptlaststufe 3a überführt. Der Eingang des Stutzens zu der Pumpe 5d liegt hierbei im unteren Bereich des Vorversäuerers 2, da hier während der Flotation keine nicht ausreichend vorversäuerten Feststoffe vorliegen. Der Stutzen kann aber auch im mittleren oder oberen Bereich liegen, wenn nicht flotiert sondern sedimentiert wird.

Ein optional vorgesehenes Rührwerk 6, 7 ist beim Überführen vorteilhafterweise ausgeschaltet.

Mit dem oben beschriebenen Verfahren ist es z. B. möglich, die mittlere Aufenthaltsdauer von flüssigen Stoffen A in der Vorversäuerung 2 auf etwa 5 bis 15, vorteilhafterweise etwa 10 Stunden einzustellen und die mittlere Aufenthaltsdauer von Feststoffen auf zwischen 30 und 150, bevorzugterweise ca. 100 Stunden, einzustellen. Dadurch ist es möglich nur ausreichend vorversäuerte Stoffe in die Hauptlaststufe 3a zu überführen und gleichzeitig jedoch gemeinsam oder einzeln sowohl flüssige als auch feste Stoffe A, B zu verarbeiten.

Es hat sich gezeigt, dass mit den obigen Verfahren eine Verwertung der Feststoffe für die Biogasgewinnung von bis zu 80 % und mehr möglich ist.

Die Vorteile in wirtschaftlicher Hinsicht und verfahrenstechnischer/betriebstechnischer Hinsicht durch Reduzierung auf eine Reaktorlinie bei der Behandlung an sich verschiedenartiger organischer Stoffe sind groß.

## Patentansprüche

1. Vorrichtung (1) zum anaeroben Fermentieren von Stoffen (A, B) mit einem Vorversäuerer (2), in dem die Stoffe (A, B) einer Vorversäuerung unterliegen,
einem Fermenter (3a, 3b), in dem die vorversäuerten Stoffe (A, B) fermentieren und Überführmittel (5a, 5b, 5c, 5d, 5e, 10, 11, 12, 14, 21) zum Überführen der Stoffe (A, B) aus dem Vorversäuerer (2) in den Fermenter (3a, 3b),
**dadurch gekennzeichnet, dass**
die Überführmittel (5a, 5b, 5c, 5d, 5e, 10, 11, 12, 14, 21) zum selektiven Überführen von ausreichend vorversäuerten Stoffen (A, B) ausgebildet sind.

2. Vorrichtung (1) nach Anspruch 1, **dadurch gekennzeichnet, dass** die Überführmittel (5a, 5b, 5c, 14) eine Entnahmeeinrichtung (5a, 5b, 5c) zur Entnahme aus dem oberen Bereich des Vorversäuerers (2) umfassen, die vorzugsweise durch einen Überlauf des Vorversäuerers (2) oder ein im oberen Bereich des Vorversäuerers (2) angeordnetes Entnahmestutzenende ausgebildet sind.

3. Vorrichtung (1) nach Anspruch 2 **dadurch gekennzeichnet, dass** die Überführmittel (5a, 5b, 5c, 14) eine Steuereinrichtung (14) für die Entnahmeeinrichtung umfassen, mit der die Entnahmeeinrichtung (5a, 5b, 5c) und vorzugsweise eine Umwälzeinrichtung wie etwa ein Rührwerk (6, 7) angesteuert werden kann.

4. Vorrichtung (1) nach Anspruch 1 oder 2, **dadurch gekennzeichnet, dass** die Überführmittel (5b, 12) ein Sieb (12) umfassen.

5. Vorrichtung (1) nach Anspruch 1, **dadurch gekennzeichnet, dass** die Überführmittel (5d, 5e, 10, 11, 14, 21) eine Flotationseinrichtung (10, 11, 21) und eine Entnahmeeinrichtung (5d, 5e) im unteren Bereich des Vorversäuerers (2) umfassen.

6. Vorrichtung (1) nach Anspruch 5, **dadurch gekennzeichnet, dass** die Überführmittel (5d, 5e, 10, 11, 14, 21) eine Steuereinrichtung (14) für die Entnahmeeinrichtung (5d, 5e) umfassen, mit der die Entnahmeeinrichtung (5d, 5e) und vorzugsweise die Flotationseinrichtung (10, 11, 21) angesteuert werden kann.

7. Vorrichtung (1) nach einem der Ansprüche 1 bis 6, **dadurch gekennzeichnet, dass** eine mechanische Vorbehandlungsvorzerkleinereinrichtungseinrichtung (25) zum Aufschließen/Zerkleinern von wenigstens einem Teil der Stoffe (A, B) vorgesehen ist.

8. Verfahren zum anaeroben Fermentieren von Stoffen (A, B) mit
einer Vorversäuerung, bei der die Stoffe (A, B) mit einem Vorversäuerer (2) vorversäuert werden,
einem Fermentieren, bei dem die vorversäuerten Stoffe (A, B) in einem Fermenter (3a, 3b) fermentieren, und
einem Überführen, bei dem Stoffe (A, B) aus dem Vorversäuerer (2) in den Fermenter (3a, 3b) überführt werden, **dadurch gekennzeichnet, dass**
selektiv die ausreichend vorversäuerten Stoffe überführt werden.

9. Verfahren nach Anspruch 8, **dadurch gekennzeichnet, dass** das Überführen ein sich Setzenlassen der Stoffe im Vorversäuerer (2) und ein anschließendes Entnehmen von Stoffen aus einem oberen Bereich des Vorversäuerers (2) umfassen.

10. Verfahren nach Anspruch 8 oder 9, **dadurch gekennzeichnet, dass** die Stoffe beim Überführen durch ein Sieb (12) geführt werden.

11. Verfahren nach Anspruch 8, **dadurch gekennzeichnet, dass** das Überführen eine Flotation und ein zumindest teilweise gleichzeitiges Überführen aus dem unteren Bereich des Vorversäuerers (2) umfasst.

12. Verfahren nach einem der Ansprüche 8 bis 11, **dadurch gekennzeichnet, dass** die Stoffe (A, B) Flüssigkeiten (A) und Feststoffe (B) umfassen.

13. Verfahren nach einem der Ansprüche 8 bis 12, **dadurch gekennzeichnet, dass** wenigstens ein Teil der Stoffe (A, B), insbesondere die Feststoffe (B) vorbehandlet, vorzugsweise mechanisch vorzerkleinert werden, bevor sie in den Vorversäuerer (2) gegeben werden.
